# EUROPÄISCHE PATENTSCHRIFT

(11) **EP 2 022 448 B1**
(45) Veröffentlichungstag und Bekanntmachung des Hinweises auf die Patenterteilung: **26.01.2011**
(21) Anmeldenummer: 08012903.4
(22) Anmeldetag: 17.07.2008
(51) Int. Cl.: A61F 2/38

(54) **Künstliches Gelenk und ein zu diesem Einsatz bestimmtes Gelenkteil**
Artificial joint and a joint part for this purpose
Prothèse en plastique et élément d'articulation correspondant

(30) Priorität: 07.08.2007 DE 102007037154
(43) Veröffentlichungstag der Anmeldung: 11.02.2009
(73) Patentinhaber: Aequos Endoprothetik Gmbh, 82166 Gräfelfing (DE)
(72) Erfinder: Nägerl, Hans, Prof. Dr., 37130 Gleichen (DE)
(74) Vertreter: Scheffler, Jörg

(56) Entgegenhaltungen:
- EP-A- 0 346 183
- EP-A- 0 600 806
- EP-A- 0 749 734
- WO-A-92/08424
- WO-A-98/46171
- US-A1- 2004 243 245

## Beschreibung

Die Erfindung betrifft ein künstliches Gelenk als Endoprothese für ein menschliches Gelenk, insbesondere Kniegelenk, bestehend aus mindestens zwei Gelenkteilen, wobei jedes Gelenkteil zwei Funktionsflächen aufweist, wobei die beiden Funktionsflächen jedes Gelenkteils sphäroidal geformt und in der proximal-distalen Zuordnung konvex-konkav, konkav-konvex oder konvex-konvex geformt sind, und die Funktionsflächen um jeweils eine Schwenkachse schwenkbeweglich sind und derart geformt sind, dass sich bei der Extension/Flexion des Gelenks eine Kombination aus einer Roll- und Gleitbewegung einstellt, wobei das künstliche Gelenk eine durch einen Vorsprung, welcher dem ersten Gelenkteil zugeordnet ist, sowie eine Ausnehmung, welche dem zweiten Gelenkteil zugeordnet ist, gebildete Ausformung hat, welche die Relativposition der beiden Gelenkteile in der Transversalebene festlegt und zugleich einen Anschlag für eine Gleitbewegung der Gelenkteile relativ zueinander bildet. Weiterhin betrifft die Erfindung ein zum Einsatz in ein solches künstliches Gelenk bestimmtes Gelenkteil.

Ein solches künstliches Gelenk dient beispielsweise als Endoprothese für das menschliche Kniegelenk, welches aufgrund der Inkongruenz seiner knöchernen Partner - Femurcondylen, Tibiaplateau und Patellarückfläche - einer sekundären Stabilisierung bedarf. Zu den sekundären Stabilisatoren gehören als passiv wirkende Strukturen neben den knöchernen Anteilen der Kapsel-Band-Apparat und die Menisken. Aktiv tragen die Muskeln und Sehnen zur Stabilität bei. Diese sekundären Stabilisatoren begrenzen das Bewegungsausmaß des Gelenkes. Der hierdurch entstehende Stabilitätsgrad ist für das Kniegelenk von besonderer Bedeutung.

Als Hauptbewegungsebene gilt die Extension/Flexion, diese stellt eine Kombination aus einer Roll- und Gleitbewegung der Femurcondylen über das Tibiaplateau dar. Das vordere und hintere Kreuzband bilden zusammen den zentralen Pfeiler des Kniegelenkes. Sie stabilisieren die Roll- und Gleitbewegung des Kniegelenkes und verhindern, dass sich die Gelenkflächen gegeneinander verschieben.

Durch plötzliche unkontrollierte Verdrehbewegungen des Kniegelenkes, die von der Muskulatur nicht abgefangen werden, kann es zum Zerreißen der Kreuzbänder kommen. Meist ist das vordere Kreuzband betroffen, Verletzungen des hinteren Kreuzbandes sind eher selten.

In der Praxis ist in solchen Fällen oftmals ein operativer Kreuzbandersatz erforderlich. Dabei wird bei einem wenig traumatisierenden Operations-Verfahren eine "innere Verankerung" einer Kreuzband-Ersatzsehne im Gelenk vorgenommen. Durch einen kleinen Schnitt am Schienbeinkopf wird mit einem speziellen Instrument die Sehne eines Adduktorenmuskels unter der Haut von dem Muskel abgetrennt.

Diese ca. 30 cm lange Sehne wird dann vierfach aufeinander gelegt, wodurch eine Ersatzsehne für das Kreuzband entsteht. Diese Sehne muss anatomisch korrekt anstelle des gerissen Kreuzbandes in das Gelenk eingezogen werden. Dazu werden mit Hilfe von Zielgeräten Bohrkanäle durch den Schienbeinkopf und den Oberschenkelknochen angelegt. Die Sehne wird dann durch die beiden Bohrkanäle in das Gelenk eingezogen. Zur stabilen Fixation der Sehne im Knochenkanal gibt es verschiedene Verfahren. Die Entscheidung, welches Verfahren angewandt wird, hängt von individuellen anatomischen Voraussetzungen des Patienten, wie Länge und Dicke des Bandes, ab.

Ein gattungsgemäßes Gelenk ist beispielsweise durch die WO 98/46171 A1 bekannt, bei der die relative Gleitbewegung der Gelenkteile zueinander in den Extremlagen der Winkelstellungen beschränkt ist.

Ein als eine Kniegelenkprothese ausgeführtes Gelenk ist beispielsweise durch die DE 696 23 861 T2 offenbart, bei der eine dem tibialen Gelenkteil zugeordnete mittlere Rippe in eine Höhlung des fermoralen Gelenkteils eingreift. In der Strecklage liegt die Wand der Höhlung gegen die Vorderseite der Rippe an. Daraus ergibt sich ein allein in der Strecklage, also in einer einzigen Position, wirksamer Anschlag.

Weiterhin ist ein Gelenk beispielsweise durch die EP 07 34 700 A 2 bekannt. Bei dieser ist die Gelenkgeometrie der Funktionsflächen zueinander in jeder der beiden Ebenen durch eine Gelenkkette mit zwei Gelenkachsen bestimmt, die durch die Rotationszentren der Funktionsflächen mit den Radien der jeweils zugehörigen Schnittkonturen verlaufen, wobei eine femurseitige, also gelenkkopfseitige Verbindung der Mittelpunkte der Gelenkköpfe einem Gestell und eine tibiaseitige, also gelenkpfannenseitige Verbindung der Mittelpunkte der Gelenkpfannen einer Koppel einer die vier Achsen aufweisenden Viergelenkkette entsprechen.

Durch die DE 196 46 891 A1 ist ein künstliches Gelenk, insbesondere eine Endoprothese zum Ersatz natürlicher Gelenke, bestehend aus mindestens zwei künstlichen Gelenkteilen mit gekrümmten Artikulationsflächen, bekannt, wobei auf jeder der Artikulationsflächen eine gekrümmte Kontaktlinie ausgebildet ist. Die gekrümmte Kontaktlinie einer der Artikulationsflächen ist ein Teil einer elliptischen Schnittkontur eines ersten Zylinders oder Kegels mit dem Zylinderradius bzw. dem Kegelwinkel. Die andere Kontaktlinie ergibt sich als Gegenspur eines auf dem ersten Zylinder bzw. ersten Kegel abrollenden oder gleitenden zweiten Zylinders bzw. zweiten Kegels mit dem Zylinderradius bzw. mit dem Kegelwinkel. Die Artikulationsflächen weisen aus einer Vielzahl von geraden Berührungslinien gebildete Regelflächen auf. Diese Regelflächen schließen sich gegenseitig an die Kontaktlinien einander gegenüberliegend an und die Berührungslinien sind jeweils die Verbindungslinien zwischen einem momentanen Kontaktpunkt der Kontaktlinien und einem momentan gemeinsamen Punkt bzw. dem Momentanpol der jeweiligen Bewegungssysteme in einer Referenzebene bzw. einer Referenzkugel im bewegten/unbewegten System.

Auch die DE 195 21 597 A1 betrifft ein künstliches Gelenk, insbesondere eine Endoprothese zum Ersatz natürlicher Gelenke, bestehend aus mindestens zwei künstlichen Gelenkteilen mit gekrümmten Artikulationsflächen, wobei auf jeder der Artikulationsflächen eine kreisbogenförmige Kontaktlinie ausgebildet ist, die jeweils ein Teilabschnitt eines in einer Ebene liegenden Kontaktkreises mit einem Mittelpunkt ist. Die Artikulationsflächen sind derart als Paar zueinander angeordnet, dass die Kontaktlinien aufeinander abrollen können sowie senkrecht zu der Ebene der Kontaktkreise durch deren Mittelpunkt verlaufende Achsen sich in einem Schnittpunkt schneiden. Einseitig an die Kontaktlinien sind aus einer Vielzahl von geraden Berührungslinien gebildete Regelflächen angeformt, wobei die Berührungslinien auf momentanen Verbindungslinien der während der Abrollbewegung auftretenden momentanen Kontaktpunkte mit den momentanen Schnittpunkten liegen, welche sich durch eine Schwenkbewegung der Kontaktlinien mit einer Winkelgeschwindigkeit um eine gemeinsame Tangente der Kontaktlinien durch die momentanen Kontaktpunkte ergeben.

Die EP 600 806 A1 offenbart auch bereits eine dreiteilige Knieprothese mit einem femoralen und einem tibialen Implantat, wobei das femorale Implantat eine Einkerbung aufweist, durch die zwei auseinander laufende und durch eine Trochlea verbundene Kondylenauflageschuhe umschrieben werden, wobei das femorale Implantat eine Form besitzt, die sich aus der Kombination der folgenden Merkmale ergibt, dass nämlich der innere und der äußere Kondylenschuh in sagittaler Ebene unterschiedliche Krümmungsradien haben, die Auflage- und Gleitflächen des inneren und äußeren Kondylenschuhs in frontaler Ebene Unterschiede in Breite und Querschnitt aufweisen, der innere und äußere Kondylenschuh im hinteren Teil unterschiedliche Rollamplituden besitzen, die Trochlea in frontaler und sagittaler Ebene eine Fläche mit dem Querschnitt eines geometrischen Torus begrenzt, wobei sie auf der Außenseite in anatomischer Weise angehoben ist, die Spanne der Auflage- und Gleitflächen der Kondylenschuhe im Vergleich zu den anatomischen Verhältnissen erhöht ist und die Kondylenschuhe abgeflacht sind, um die Druckverteilung zu begünstigen.

Aus der deutschen Patentanmeldung DE 39 08 958 A1 ist bereits ein zum Ersatz menschlicher Gelenke bestimmtes künstliches Gelenk bekannt, welches aus zwei Gelenkteilen mit beweglichen Funktionsflächen besteht. Die Krümmungsverhältnisse der eine kreisförmige Schnittkontur aufweisenden Funktionsflächen sind zueinander konvex-konvex, konvex-konkav oder konkav-konkav und die Gelenkgeometrie ist durch eine Gelenkkette mit zwei Gelenkachsen (dimere Gelenkkette) bestimmt, die durch die Rotationszentren der Funktionsflächen verlaufen. Hierbei sind die Gelenkflächen kugelförmig ausgebildet, sodass eine Gelenkbewegung mit fünf Freiheitsgraden möglich ist.

Ausgehend von diesem Stand der Technik liegt nun der Erfindung die Aufgabe zugrunde, eine Möglichkeit zu schaffen, ein künstliches Gelenk zu schaffen, welches in optimaler Weise neben der an sich bekannten Gelenkfunktion auch Teilfunktionen des Bandapparates, insbesondere des Kreuzbandes, erfüllt. Weiterhin liegt der Erfindung die Aufgabe zugrunde, einen künstlichen Gelenkteil zum Einsatz in ein solches künstliches Gelenk zu schaffen.

Die erstgenannte Aufgabe wird erfindungsgemäß mit einem künstlichen Gelenk gemäß den Merkmalen des Anspruchs 1 gelöst. Die weitere Ausgestaltung der Erfindung ist den Unteransprüchen 2 bis 10 zu entnehmen.

Erfindungsgemäß ist also ein künstliches Gelenk vorgesehen, bei dem in Abhängigkeit von der Winkelstellung der Gelenkteile eine jeweilige posteriore Position in der Sagittalebene derart bestimmt ist, dass ein posteriores Abgleiten insbesondere des distalen Gelenkteils in jeder Winkelstellung auf einen vorbestimmten Betrag beschränkt oder ausgeschlossen ist. Hierdurch wird in einfacher Weise zu jeder Beugeposition ein posteriores Verschieben des einen Gelenkteils begrenzt oder verhindert und anatomisch korrekt die Funktion des hinteren Kreuzbandes ersetzt. Der Erfindung liegt dabei die Erkenntnis zugrunde, dass eine durch die Rollbewegung definierte Ablaufstrecke vorteilhaft zugleich auch für die Festlegung der posterioren Position genutzt werden kann, wenn die Ausnehmung und der Vorsprung in jeder Beugeposition zugleich eine vorbestimmte posteriore Position oder eine beschränkte Verschiebbarkeit festlegen, insbesondere also eine posteriore Verlagerung des der Tibia zugeordneten Gelenkteils ausgeschlossen oder auf einen bestimmten Betrag beschränkt ist. Ergänzend kann die Ausformung gemeinsam mit dem eingreifenden Vorsprung am Anfangsbereich der Beugung auch die Funktion des vorderen Kreuzbandes aufnehmen, sodass zusätzliche Hilfsmittel für die Gelenkstabilisierung nicht erforderlich sind. Dabei legt der Stabilitätsbereich des natürlichen Bandapparates die Toleranz in der Relativbeweglichkeit des Vorsprungs gegenüber der Ausformung fest.

Dabei ist es besonders vorteilhaft, wenn der Betrag einer möglichen posterioren Verlagerung in Abhängigkeit des Verlustes des natürlichen Bandapparates bestimmt und/oder einstellbar ist. Hierdurch wird also in der Praxis eine vergleichsweise große posteriore Verlagerung zugelassen, wenn Fehlfunktion des Bewegungsablaufes im Wesentlichen durch den natürlichen Bandapparat vermieden bzw. behoben werden können. Zu diesem Zweck ist also der Spalt zwischen der Ausnehmung und dem Vorsprung bei weitgehend vorhandenem natürlichem Bandapparat vergleichsweise groß bemessen. Beispielsweise kann bei einem Verlust der Seitenbänder eine starke Führung durch einen geringen Spalt zwischen der Ausnehmung und dem Vorsprung bis hin zu einem formschlüssigen (constraint) Prothesendesign realisiert werden.

Eine besonders vorteilhafte Ausführungsform der vorliegenden Erfindung wird dadurch erreicht, dass durch die Ausformung ein von dem Flexionswinkel des Gelenkes abhängiger posteriorer Anschlag für den der Tibia zugeordneten Gelenkteil gebildet ist, um so die posteriore Verschiebung zuverlässig zu beschränken oder zu verhindern. Die Ausformung begrenzt dabei zugleich eine Verschiebung des der Tibia zugeordneten Gelenkteils nach posterior wie auch eine in dieser Flexionsstellung unerwünschte Gleitbewegung.

Demgegenüber ist es auch besonders praxisgerecht, wenn gemäß einer weiteren Ausgestaltung der vorliegenden Erfindung im Bereich einer relativen Gleitbewegung der beiden Gelenkteile die posteriore Position unabhängig von dem Flexionswinkel konstant ist. Hierdurch wird im Gegensatz zu der im Anfangsbereich der Flexion erwünschten Rollphase, in welcher durch die Zusammenwirkung der Ausnehmung mit dem Vorsprung einem bestimmten Flexionswinkel eine bestimmte posteriore Position zugeordnet wird, ein veränderlicher Flexionswinkel bei unveränderter posteriorer Relativposition der beiden Gelenkteile ermöglicht.

Die posteriore Blockade könnte bei verändertem Flexionswinkel in diskreten Abstufungen erfolgen. Demgegenüber ist es jedoch besonders Erfolg versprechend, wenn im Bereich einer relativen Rollbewegung der beiden Gelenkteile die Veränderung der posterioren Position in Abhängigkeit von dem Flexionswinkel der Gelenkteile insbesondere linear bestimmt ist. Hierdurch wird also jedem Flexionswinkel eine bestimmte posteriore Position des der Tibia zugeordneten Gelenkteils erreicht. Die Beugebewegung wird dabei durch die posteriore Blockade in keiner Weise beeinträchtigt.

Eine besonders Erfolg versprechende Ausgestaltung der vorliegenden Erfindung wird dadurch erreicht, dass die Ausnehmung oder der Vorsprung in der Transversalebene eine nach posterior abnehmende laterale Erstreckung bzw. Breite aufweist, sodass aufgrund der Rollbewegung der Gelenkteile der insbesondere der Tibia zugeordnete Vorsprung in die insbesondere dem Femur zugeordnete Ausnehmung eingreift. Der Rollbewegung folgend nimmt die laterale Erstreckung, also die Breite der Ausnehmung, dabei derart ab, dass die Verlagerung ausschließlich aufgrund der relativen Winkelstellung der Gelenkteile zueinander ermöglicht wird.

Die Ausformung könnte tropfenförmig, schweifförmig oder wulstartig ausgeführt sein. Besonders einfach ist es hingegen auch, wenn die Ausformung in der Transversalebene einen nach Art einer Schwalbenschwanzverbindung ausgeführten Vorsprung mit insbesondere gerade verlaufenden Flanken aufweist, um so eine zuverlässige Führung zu erreichen, die lediglich geringfügigen Änderungen des erfindungsgemäßen künstlichen Gelenkes gegenüber an sich bekannten Gelenken erfordert. Insbesondere kann also die Funktionsweise im Hinblick auf die zu realisierende Roll-Gleitbewegung mit der Ausformung problemlos kombiniert werden.

Hierbei ist es besonders sinnvoll, wenn die Ausnehmung zwischen den konvexen Funktionsflächen des insbesondere dem Femur zugeordneten Gelenkteils gebildet ist, sodass beispielsweise auch unterschiedliche Gelenkteile kombinierbar sind, die wahlweise eine Ausformung realisieren, sodass bei entsprechender Indikation ein späterer Austausch eines Gelenkteils mit einem vergleichsweise geringen Aufwand realisierbar ist.

Grundsätzlich eignet sich das so geschaffene künstliche Gelenk für den Ersatz unterschiedlicher menschlicher Gelenke. Besonders Erfolg versprechend ist hingegen eine Anwendung, bei welcher der Vorsprung dem tibialen Gelenkteil und die Ausnehmung dem femoralen Gelenkteil zugeordnet ist, um so den Vorsprung sowie die Ausnehmung jeweils zwischen den beiden Funktionsflächen desselben Gelenkteils realisieren zu können. Dies wird beispielsweise erreicht, durch einen tropfen- und schweifförmigen Ausschnitt mit Führungsnutzen der inneren Abhänge des femoralen Gelenkteils und durch eine entsprechende tropfenförmige, horizontale und schräg angepasste vertikale Umformung des der Tibia zugeordneten Gelenkteils.

Eine andere besonders sinnvolle Weiterbildung wird dadurch erreicht, dass eines der Gelenkteile eine insbesondere nutenförmige Ausnehmung hat, in welche ein Vorsprung des anderen Gelenkteils eingreift, um so das Abheben der Funktionsflächen verschiedener Gelenkteile zu verhindern.

Dabei ist es zudem besonders vorteilhaft, wenn der Vorsprung oder die Ausnehmung oder zumindest eines der Gelenkteile austauschbar oder nachrüstbar an dem Gelenk fixierbar ist, um so eine optimale Anpassung an verschiedene, sich insbesondere verändernde Anforderungen erfüllen zu können. Sofern also beispielsweise die posteriore Blockade nicht erwünscht ist, kann problemlos ein anderes Gelenkteil, welches die entsprechende Ausnehmung oder den entsprechenden Vorsprung nicht aufweist, vorgesehen werden.

Die zweitgenannte Aufgabe wird durch ein künstliches Gelenkteil mit einem Vorsprung oder einer Ausnehmung zum Einsatz in ein künstliches Gelenk nach zumindest einem der vorhergehenden Ansprüche realisiert, welches bedarfsweise beispielsweise auch zum Austausch eines einzelnen Gelenkteils eines bereits eingesetzten künstlichen Gelenks dient. Dabei kann beispielsweise der dem Femur zugeordnete Gelenkteil mit verschiedenen, der Tibia zugeordneten Gelenkteilen kombiniert werden, die in Abhängigkeit der jeweiligen Indikation einsetzbar sind.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: einen tibialen Gelenkteil mit zwei einen Vorsprung einschließenden Funktionsflächen in einer perspektivischen Ansicht;
- Fig. 2: eine Zuordnung des in der Figur 1 gezeigten tibialen Gelenkteils und eines femoralen Gelenkteils in einer stark gebeugten Position in einer perspektivischen Ansicht;
- Fig. 3: eine Zuordnung der in der Figur 2 gezeigten tibialen und femoralen Gelenkteile in einer gestreckten Position in einer rückwärtigen Ansicht von schräg oben;
- Fig. 4: einen Ausschnitt einer Seitenfläche A der tibialen und femoralen Gelenkteile der Figur 3 in einer vergrößerten Schnittdarstellung;
- Fig. 5: einen Ausschnitt einer Seitenfläche B der tibialen und femoralen Gelenkteile der Figur 3 in einer vergrößerten Schnittdarstellung.

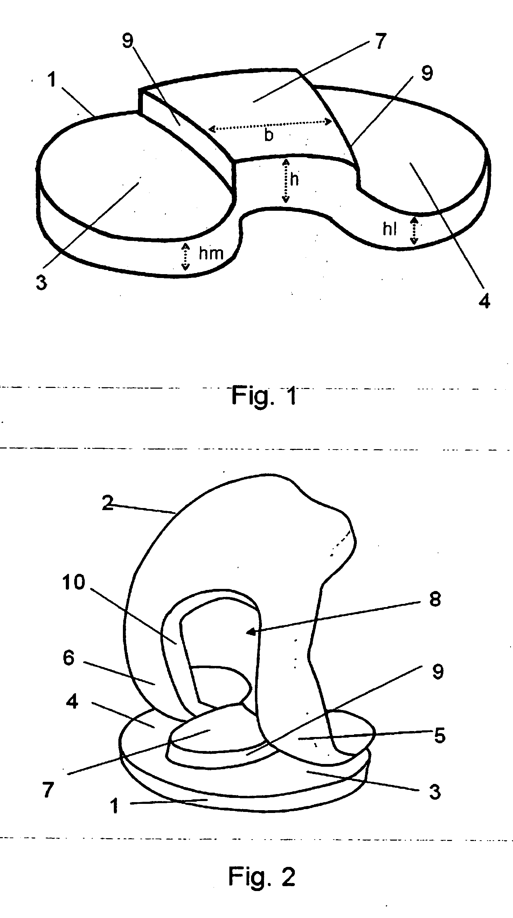

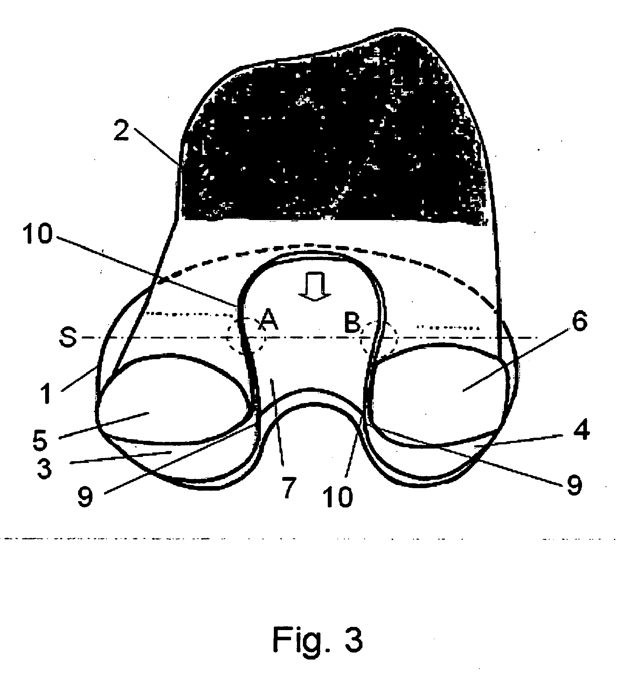

Das erfindungsgemäße künstliche Gelenk wird nachstehend anhand der Figuren 1 bis 5 näher dargestellt, wobei in der Figur 1 eine perspektivische Ansicht eines tibialen Gelenkteils 1 und in Figur 2 eine perspektivische Ansicht einer Zuordnung des tibialen Gelenkteils 1 und eines femoralen Gelenkteils 2 als Endoprothese für ein menschliches Kniegelenk in einer stark gebeugten Position gezeigt ist. Jedes Gelenkteil 1 weist zwei Funktionsflächen 3, 4 auf, die jeweils mit einer Funktionsfläche 5, 6 des anderen Gelenkteils 2 zusammenwirken, wobei die beiden Funktionsflächen 3, 4, 5, 6 jedes Gelenkteils 1, 2 sphäroidal geformt und in der proximal-distalen Zuordnung konvex-konkav, konkav-konvex oder konvex-konvex geformt sind. Die Funktionsflächen 3, 4, 5, 6 sind derart geformt, dass sich bei der Extension/Flexion des Gelenks eine Kombination aus einer Roll- und Gleitbewegung einstellt. Um in jeder Beugeposition eine posteriore Blockade entsprechend dem hinteren Kreuzband zu realisieren und darüber hinaus im Stand eine posteriore und zugleich eine anteriore Blockade bei geringem Beugewinkel bis zu 10° entsprechend der Funktion des vorderen Kreuzbandes zu ermöglichen, hat das künstliche Gelenk eine durch einen Vorsprung 7, welcher dem ersten Gelenkteil 1 zugeordnet ist, sowie durch eine Ausnehmung 8, welche dem zweiten Gelenkteil 2 zugeordnet ist, gebildete Ausformung. Aufgrund dieser Ausformung wird die Relativposition der beiden Gelenkteile 1, 2 in der Transversalebene festgelegt und zugleich ein Anschlag für eine Gleitbewegung der Gelenkteile relativ zueinander gebildet. Die Ausnehmung 8 sowie der Vorsprung 7 weisen hierzu in der Transversalebene eine nach posterior abnehmende laterale Erstreckung nach Art einer abgerundeten Schwalbenschwanzverbindung auf. Die Ausnehmung 8 sowie der Vorsprung 7 weisen jeweils eine Kontaktlinie 9, 10 auf, auf der sich Abhängigkeit der Beugung zumindest zwei als posteriore Blockade wirkende Kontaktpunkte oder auf der sich in Abhängigkeit der Beugung bei posteriorer Verschiebung des Gelenkteils 1 zumindest zwei als posteriore Blockade wirkende Kontaktpunkte ergeben, die zueinander in Abhängigkeit der Beugung einen Abstand b einschließen. Durch die Differenz der Höhe h des Vorsprungs 7 gegenüber der Höhe hm der Funktionsfläche 3 und der Höhe hl der Funktionsfläche 4 können zusätzlich auch die Funktionen der Seitenbänder erfüllt oder unterstützt werden.

Ergänzend zeigt die Figur eine Zuordnung der in der Figur 2 gezeigten tibialen und femoralen Gelenkteile 1, 2 in einer gestreckten Position aus einer rückwärtigen Perspektive von schräg oben. Wie deutlich zu erkennen, ist ein Verrutschen des tibialen Gelenkteils 1 in Relation zu dem femoralen Gelenkteil 2 aufgrund der konischen Form der auf der Seitenfläche gebildeten Kontaktlinie 9 des tibialen Gelenkteils 1 an dem Vorsprung 7 und der Kontaktlinie 10 des femoralen Gelenkteils 2 ausgeschlossen. Hierdurch wird eine unerwünschte Verlagerung der Funktionsflächen 3, 4, 5, 6 vermieden.

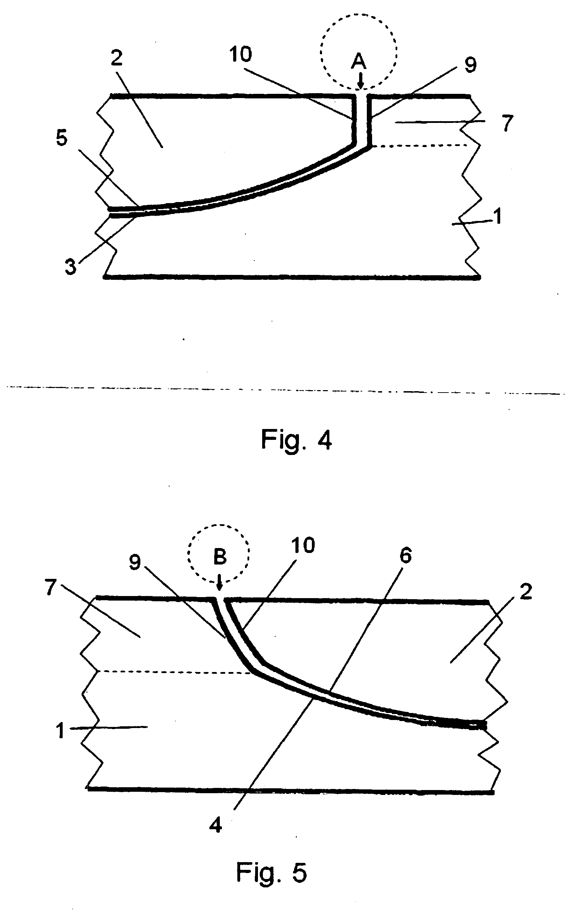

Mögliche Gestaltungen der auf den Seitenflächen A und B gebildeten Kontaktlinie 9 des tibialen Gelenkteils 1 an dem Vorsprung 7 entlang der Schnittlinie S senkrecht zu der Fläche des tibialen Gelenkteils 1 und der Kontaktlinie 10 des femoralen Gelenkteils 2 werden zusätzlich auch in den Figuren 4 und 5 gezeigt, die einen jeweiligen vergrößerten Ausschnitt der Gelenkteile 1, 2 in einer Schnittdarstellung zeigen. Zu erkennen ist in der Figur 4 der zu der Oberfläche des tibialen Gelenkteils 1 senkrechte Verlauf der Seitenfläche A. Demgegenüber weist die Seitenfläche B zwischen der Kontaktlinie 9 des Vorsprungs 7 auf dem tibialen Gelenkteil 1 und der Kontaktlinie 10 des femoralen Gelenkteils 2 eine abgerundete Beschaffenheit auf, wie dies aus der Figur 5 ersichtlich ist.

## Patentansprüche

1. Künstliches Gelenk als Endoprothese für ein menschliches Gelenk, insbesondere Kniegelenk, bestehend aus mindestens zwei Gelenkteilen (1, 2), wobei jedes Gelenkteil (1, 2) zwei Funktionsflächen (3, 4) aufweist, wobei die beiden Funktionsflächen (3, 4, 5, 6) jedes Gelenkteils (1, 2) sphäroidal und in der proximal-distalen Zuordnung konvex-konkav, konkav-konvex oder konvex-konvex geformt sind und die Funktionsflächen (3, 4, 5, 6) um jeweils eine Schwenkachse schwenkbeweglich sind und derart geformt sind, dass sich bei der Extension/Flexion des Gelenks eine Kombination aus einer Roll- und Gleitbewegung einstellt, wobei das künstliche Gelenk eine durch einen Vorsprung (7), welcher dem ersten Gelenkteil (1) zugeordnet ist, sowie eine Ausnehmung (8), welche dem zweiten Gelenkteil (2) zugeordnet ist, gebildete Ausformung hat, welche die Relativposition der beiden Gelenkteile (1, 2) in der Transversalebene festlegt und zugleich einen Anschlag für eine Gleitbewegung der Gelenkteile (1, 2) relativ zueinander bildet, wobei in Abhängigkeit von der Extension/Flexion der Gelenkteile (1, 2) eine jeweilige posteriore Position derart bestimmt ist, dass ein posteriores Abgleiten insbesondere des distalen Gelenkteils (1) in jeder Winkelstellung auf einen vorbestimmten Betrag beschränkt oder ausgeschlossen ist, und durch die Ausformung ein von dem Flexionswinkel des Gelenks abhängiger posteriorer Anschlag gebildet ist, **dadurch gekennzeichnet, dass** die Ausnehmung (8) und der Vorsprung (7) in der Transversalebene eine nach posterior abnehmende laterale Erstreckung (Abstand b) aufweisen.

2. Gelenk nach Anspruch 1, **dadurch gekennzeichnet, dass** der Betrag einer möglichen posterioren Verlagerung in Abhängigkeit des Verlusts des natürlichen Bandapparats einstellbar ist.

3. Gelenk nach den Ansprüchen 1 oder 2, **dadurch gekennzeichnet, dass** im Bereich einer relativen Gleitbewegung der beiden Gelenkteile (1, 2) die posteriore Position unabhängig von dem Flexionswinkel konstant ist.

4. Gelenk nach zumindest einem der vorhergehenden Ansprüche, **dadurch gekennzeichnet, dass** im Bereich einer relativen Rollbewegung der beiden Gelenkteile (1, 2) die Veränderung der posterioren Position in Abhängigkeit von dem Flexionswinkel der Gelenkteile (1, 2) linear bestimmt ist.

5. Gelenk nach zumindest einem der vorhergehenden Ansprüche, **dadurch gekennzeichnet, dass** die Ausformung in der Transversalebene einen nach Art einer Schwalbenschwanzverbindung ausgeführten Vorsprung (7) mit insbesondere gerade verlaufenden Flanken aufweist.

6. Gelenk nach zumindest einem der vorhergehenden Ansprüche, **dadurch gekennzeichnet, dass** die Ausnehmung (8) zwischen den konvexen Funktionsflächen (5, 6) des insbesondere dem Femur zugeordneten Gelenkteils (2) gebildet ist.

7. Gelenk nach zumindest einem der vorhergehenden Ansprüche, **dadurch gekennzeichnet, dass** der Vorsprung (7) dem tibialen Gelenkteil (1) und die Ausnehmung (8) dem femoralen Gelenkteil (2) zugeordnet ist.

8. Gelenk nach zumindest einem der vorhergehenden Ansprüche, **dadurch gekennzeichnet, dass** eines der Gelenkteile (2) eine nutenförmige Ausnehmung (8) hat, in welche ein Vorsprung (7) des anderen Gelenkteils (1) eingreift.

9. Gelenk nach zumindest einem der vorhergehenden Ansprüche, **dadurch gekennzeichnet, dass** der Vorsprung (7) und/oder die Ausnehmung (8) austauschbar und/oder nachrüstbar an dem jeweiligen Gelenkteil (1, 2) fixierbar sind.

10. Gelenk nach zumindest einem der vorhergehenden Ansprüche, **dadurch gekennzeichnet, dass** zumindest eines der Gelenkteile (1, 2) austauschbar und/oder nachrüstbar an dem Gelenk fixierbar ist.

11. Künstliches Gelenkteil mit einem Vorsprung (7) oder einer Ausnehmung (8) zum Einsatz in ein künstliches Gelenk nach zumindest einem der vorhergehenden Ansprüche.

## Claims

1. Artificial joint as an endoprosthesis for a human joint, more particularly a knee joint, consisting of at least two joint parts (1, 2), each joint part (1, 2) having two functional surfaces (3, 4), with the two functional surfaces (3, 4, 5, 6) of each joint part (1, 2) being spheroidal and formed convex-concave, concave-convex or convex-convex in the proximal-distal classification and the functional surfaces (3, 4, 5, 6) being able to undergo pivot motion about respectively one pivot axis and being formed such that when the joint extends/flexes a combination of rolling and sliding motion is set, with the artificial joint having a form formed by a projection (7), which is associated with the first joint part (1), and a recess (8), which is associated with the second joint part (2), which form fixes the relative position of the two joint parts (1, 2) in the transversal plane and at the same time forms a stop for a sliding motion of the joint parts (1, 2) relative to one another, with a respective posterior position being determined as a function of the extension/flexion of the joint parts (1, 2) such that posterior sliding off, in particular of the distal joint part (1), is limited to a predetermined value or prevented in each angular position, and the form forms a posterior stop depending on the flexion angle of the joint, **characterized in that** the recess (8) and the projection (7) have a lateral extent (distance b) in the transversal plane that reduces in the posterior direction.

2. Joint according to Claim 1, **characterized in that** the magnitude of a possible posterior displacement can be set as a function of the loss of the natural ligament apparatus.

3. Joint according to Claim 1 or 2, **characterized in that** the posterior position is constant, independent of the flexion angle, in the region of a relative sliding motion of the two joint parts (1, 2).

4. Joint according to at least one of the preceding claims, **characterized in that** the change in the posterior position is determined linearly, as a function of the flexion angle of the joint parts (1, 2), in the region of a relative rolling motion of the two joint parts (1, 2).

5. Joint according to at least one of the preceding claims, **characterized in that** the form in the transversal plane has a projection (7), formed like a dovetail joint, with, in particular, straight flanks.

6. Joint according to at least one of the preceding claims, **characterized in that** the recess (8) is formed between the convex functional surfaces (5, 6) of the joint part (2) associated with, in particular, the femur.

7. Joint according to at least one of the preceding claims, **characterized in that** the projection (7) is associated with the tibial joint part (1) and the recess (8) is associated with the femoral joint part (2).

8. Joint according to at least one of the preceding claims, **characterized in that** one of the joint parts (2) has a groove-like recess (8), into which a projection (7) of the other joint part (1) engages.

9. Joint according to at least one of the preceding claims, **characterized in that** the projection (7) and/or the recess (8) can be fixed to the respective joint part (1, 2) in a replaceable and/or retrofit fashion.

10. Joint according to at least one of the preceding claims, **characterized in that** at least one of the joint parts (1, 2) can be fixed to the joint in a replaceable and/or retrofit fashion.

11. Artificial joint part with a projection (7) or a recess (8) for use in an artificial joint according to at least one of the preceding claims.

## Revendications

1. Articulation artificielle en tant qu'endoprothèse pour une articulation humaine, notamment une articulation du genou, constituée d'au moins deux parties d'articulation (1, 2), chaque partie d'articulation (1, 2) présentant deux surfaces fonctionnelles (3, 4), les deux surfaces fonctionnelles (3, 4, 5, 6) de chaque partie d'articulation (1, 2) ayant une forme sphéroïdale et une association proximale-distale convexe-concave, concave-concave ou convexe-convexe, et les surfaces fonctionnelles (3, 4, 5, 6) pouvant pivoter autour d'un axe de pivotement respectif et étant formées de telle sorte que lors de l'extension/flexion de l'articulation, il s'établisse une combinaison d'un mouvement de roulement et de glissement, l'articulation artificielle ayant une formation formée par une saillie (7) qui est associée à la première partie d'articulation (1), ainsi qu'un évidement (8) qui est associé à la deuxième partie d'articulation (2), laquelle formation fixe la position relative des deux parties d'articulation (1, 2) dans le plan transversal et forme en même temps une butée pour un mouvement de glissement des parties d'articulation (1 ,2) l'une par rapport à l'autre, une position postérieure respective étant déterminée en fonction de l'extension/flexion des parties d'articulation (1, 2), de telle sorte qu'un glissement postérieur, notamment de la partie d'articulation distale (1) dans chaque position angulaire étant limitée à une valeur prédéterminée ou étant exclu, et une butée postérieure dépendant de l'angle de flexion de l'articulation étant formée par la formation, **caractérisée en ce que** l'évidement (8) et la saillie (7) présentent, dans le plan transversal, une étendue latérale diminuant vers l'arrière (distance b).

2. Articulation selon la revendication 1, **caractérisée en ce que** la valeur d'un décalage postérieur possible peut être ajustée en fonction de la perte de l'appareil ligamentaire naturel.

3. Articulation selon les revendications 1 ou 2, **caractérisée en ce que** la position postérieure est constante indépendamment de l'angle de flexion dans la région d'un déplacement de glissement relatif des deux parties d'articulation (1, 2).

4. Articulation selon au moins l'une quelconque des revendications précédentes, **caractérisée en ce que** dans la région d'un mouvement de roulement relatif des deux parties d'articulation (1, 2), la variation de la position postérieure est déterminée de manière linéaire en fonction de l'angle de flexion des parties d'articulation (1, 2).

5. Articulation selon au moins l'une quelconque des revendications précédentes, **caractérisée en ce que** la formation présente dans le plan transversal une saillie (7) réalisée à la manière d'un assemblage en queue d'aronde avec des flancs s'étendant notamment de manière rectiligne.

6. Articulation selon au moins l'une quelconque des revendications précédentes, **caractérisée en ce que** l'évidement (8) est formé entre les surfaces fonctionnelles convexes (5, 6) de la partie d'articulation (2) associée notamment au fémur.

7. Articulation selon au moins l'une quelconque des revendications précédentes, **caractérisée en ce que** la saillie (7) est associée à la partie d'articulation tibiale (1) et l'évidement (8) est associé à la partie d'articulation fémorale (2).

8. Articulation selon au moins l'une quelconque des revendications précédentes, **caractérisée en ce que** l'une des parties d'articulation (2) a un évidement en forme de rainure (8) dans lequel s'engage une saillie (7) de l'autre partie d'articulation (1).

9. Articulation selon au moins l'une quelconque des revendications précédentes, **caractérisée en ce que** la saillie (7) et/ou l'évidement (8) peuvent être fixés de manière remplaçable et/ou en rattrapage sur la partie d'articulation respective (1, 2).

10. Articulation selon au moins l'une quelconque des revendications précédentes, **caractérisée en ce qu'**au moins l'une des parties d'articulation (1, 2) peut être fixée de manière remplaçable et/ou en rattrapage sur l'articulation.

11. Partie d'articulation artificielle comprenant une saillie (7) ou un évidement (8) pour l'utilisation dans une articulation artificielle selon au moins l'une quelconque des revendications précédentes.
